# EUROPEAN PATENT SPECIFICATION

(11) **EP 2 185 069 B1**
(45) Date of publication and mention of the grant of the patent: **06.03.2019**
(21) Application number: 08798921.6
(22) Date of filing: 28.08.2008
(51) Int. Cl.: A61B 5/087

(54) **ELECTRO-PNEUMATIC ASSEMBLY FOR USE IN A RESPIRATORY MEASUREMENT SYSTEM**
ELEKTROPNEUMATISCHE BAUGRUPPE ZUR VERWENDUNG IN EINEM BEATMUNGSMESSSYSTEM
ENSEMBLE ÉLECTROPNEUMATIQUE S'UTILISANT DANS UN SYSTÈME DE MESURE RESPIRATOIRE

(30) Priority: 29.08.2007 US 968732 P; 22.08.2008 US 196358
(43) Date of publication of application: 19.05.2010
(73) Proprietor: RIC Investments, LLC., Wilmington, DE 19801-1545 (US)
(72) Inventor: SCAMPOLI, Dave, South Glastonbuyry, CT 06073 (US)
(74) Representative: van Velzen, Maaike Mathilde
(86) International application number: PCT/US2008/074698
(87) International publication number: WO 2009/029732

(56) References cited:
- US-A- 4 546 778
- US-A- 5 058 601

## Description

### TECHNICAL FIELD

The present invention pertains to an integrated electro-pneumatic assembly for use in a respiratory measurement system and a method of assembling same.

### BACKGROUND OF THE INVENTION

Respiratory flow and pressure measurements during the administration of anesthesia, in intensive care environments, and in monitoring the physical condition of athletes and other individuals prior to and during the course of training programs and other medical tests provides valuable information for assessing cardiopulmonary function and breathing circuit integrity. Many different technologies have been applied to create flow and/or pressure sensors that attempt to meet the demanding requirements of these environments.

Although various other types of flow measurement apparatus are known, differential pressure flow sensors have conventionally been used to obtain respiratory flow measurements. Pressure monitoring is typically performed to measure the pressure of gas delivered (i.e., inspired), the pressure of the exhaled gas, or both. Flow measurements provide flow rate and volume information, such as tidal volume. Pressure and flow monitoring may be used in together or in conjunction with respiratory gas measurements to assess other respiratory parameters, such as oxygen consumption, carbon dioxide elimination, and even cardiac output or pulmonary capillary blood flow.

Differential pressure flow sensors operate on the basis of Bernoulli's principle, i.e., the pressure drop across a restriction, which is also referred to as the "flow element," is proportional to the volumetric flow rate of the air. The pressure drop is measured by a differential pressure sensor. The relationship between flow and the pressure drop across the restriction or other resistance to flow is dependent upon the design of the resistance. In some differential pressure flow sensors, which are commonly termed "pneumotachs", the flow restriction creates a linear relationship between the flow and the pressure differential. Such designs include the Fleisch pneumotach, in which the restriction is comprised of many small tubes or a fine screen to ensure laminar flow and a more linear response to flow. Another physical configuration for a flow sensor is to provide is a flow restriction having an orifice the size or shape of which varies in relation to the flow.

A typical differential pressure based flow sensor includes a flow element disposed in series in a respiratory conduit. The differential pressure sensor is located at or near the flow element. Tubing connects the differential pressure sensor with the flow element such that the pressure sensor is in fluid communication with the gas flow on each side of the flow restriction. More specifically, the flow element includes a pressure pickoff port on each side of the flow restriction, and the tubing transmits the pressure at each port of each side of the differential pressure sensor. Typically, several feet of flexible, small bore, dual, or triple lumen tubing are used to connect the flow element to the differential pressure sensor.

In order to maintain performance and function in a clinical environment, respiratory measurement systems also include zeroing and purging functions, in addition to the measurement functions. Differential pressure transducers and gage pressure transducers are used for flow measurement and airway pressure measurements. Flow and airway pressure measurements are performed for periods of days to weeks in the critical care environment. However, pressure sensors drift inherently due to factors including changes in temperature. As a result, periodic zeroing is required to ensure that the flow and pressure sensor are properly calibrated. Zeroing or re-calibrating a differential pressure sensor typically necessitates exposing the two sides of the differential pressure transducer to the same pressure, usually atmospheric. Zeroing or re-calibrating a gage pressure transducer typically requires exposing the circuit side of the gage pressure transducer to atmospheric pressure. Thus, the zeroing functions typically requires the use of valves positioned between the flow element and differential and/or gage pressure transducer.

Additionally, differential pressure based flow sensors are often used in clinical environments, such as critical and intensive care units, which typically include high humidity in the flow of gas to or from the patient. The high humidity in the gas flow can lead to the condensation of moisture in the pressure transmission tubing, whether or not the pressure transmission tubing or any portion of the respiratory conduit is heated. Initially, condensation may result in a damped and distorted pressure signals and, if not cleared, can result in complete blockage of the tubing. Therefore, pressure transmission tubes are periodically purged with air from a compressed gas source or a pump in order to reduce the adverse effects of condensate on pressure and flow measurements.

The complexity associated with the valves and interconnections required for the zeroing and purging functions has resulted in conventional respiratory measurement systems being "bulky", multi-piece assemblies that are difficult and costly to assemble and have many complex pneumatic connections that must be made by hand. Additionally, the relatively large number of pneumatic connections between different components results in a greater potential for leaks at these connections. Such leaks adversely affect the measurement and lead to increased variability in the pneumatic pathway, thereby increasing the variability in the measurements particularly under loaded conditions (e.g. low compliance, higher pressures).

Given these problems with conventional respiratory measurement systems, it is desirable to provide a cost-effective easy to assemble respiratory measurement solution that addresses one or more of the following problems with conventional respiratory measurement systems: (a) eliminate the pneumatic connections that must be made manually, (b) improve long term reliability, (c) improve performance, and (d) improve inter-unit repeatability by reducing the variability between pressure transmission tubing pathways.

US 5 058 601 discloses a pulmonary function tester which provides dynamic calibration. US 4 546 778 discloses a moisture detection device for detecting respiration, and which has channels formed within a casing.

### DISCLOSURE OF THE INVENTION

Accordingly, it is an object of the present invention to provide an electro-pneumatic assembly for use in a respiratory measurement system that overcomes the shortcomings of conventional respiratory measurement systems. The invention is defined by the claims.

This assembly provides a simple, robust component for managing the pneumatics of the respiratory measurement system.

These and other objects, features, and characteristics of the present invention, as well as the methods of operation and functions of the related elements of structure and the combination of parts and economies of manufacture, will become more apparent upon consideration of the following description and the appended claims with reference to the accompanying drawings, all of which form a part of this specification, wherein like reference numerals designate corresponding parts in the various figures. It is to be expressly understood, however, that the drawings are for the purpose of illustration and description only and are not intended as a definition of the limits of the invention. As used in the specification and in the claims, the singular form of "a", "an", and "the" include plural referents unless the context clearly dictates otherwise.

### BRIEF DESCRIPTION OF THE DRAWINGS

FIG. 1 is a schematic representation of a respiratory conduit that communication with an airway of an individual and which has a pneumotach and electro-pneumatic assembly of the present invention operatively couple thereto;
FIG. 2 is a perspective view of an electro-pneumatic assembly for use in a flow/pressure measurement system;
FIG. 3 is an exploded view of the electro-pneumatic assembly in FIG. 2;
FIG. 4 is a perspective view of the housing portion of the electro-pneumatic assembly in FIG. 1;
FIG. 5 is a perspective view of the lower surface of the housing in FIG. 4;
FIG. 6 is a upper view of the upper surface of the housing in FIG. 5; and.
FIG. 7 is a lower view of the lower surface of the housing in FIG. 5.

### DETAILED DESCRIPTION OF THE EXEMPLARY EMBODIMENTS

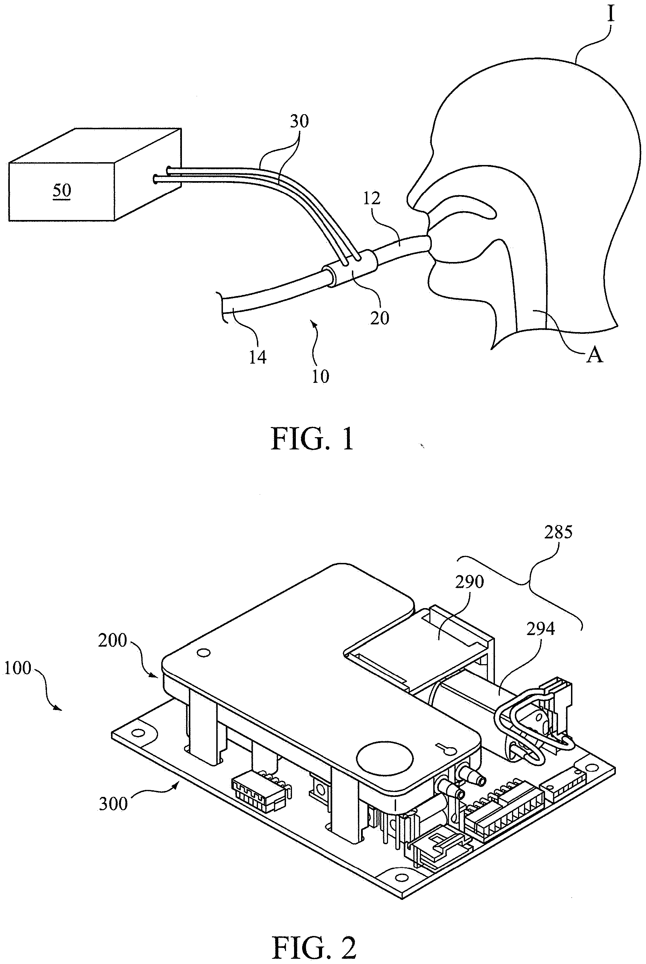

With reference to FIG. 1, a respiratory conduit 10 is depicted. In an exemplary embodiment of the present invention, respiratory conduit 10 is a breathing circuit (also referred to as a patient circuit) that includes a patient interface at one end 12. The patient interface is any device, invasive or non-invasive, that is adapted to coupled the respiratory conduit in fluid communication with an airway A of an individual 1, such as an endotracheal tube, tracheal tube, nasal mask, nasal/oral mask, or a nasal cannula. As depicted, one end 12 of respiratory conduit 10 is placed in communication with airway A, while another end 14 of the respiratory conduit opens to a source of gas to be inhaled by individual I. The present invention contemplates that the source or gas can be any gas source, such as atmosphere, an oxygen supply, a ventilator, a pressure support system (e.g., CPAP, bi-level pressure support system, auto-titration pressure support system), a source of other gas (e.g., heliox), as known in the art.

Positioned along its length, respiratory conduit 10 includes at least one airway adapter 20, which includes a flow restriction that defines a component of a pressure and/or flow sensor. Also shown in FIG. 1 is a tubing 30, which provides fluid communication between the airway adapter and a respiratory monitor 50 containing an electro-pneumatic assembly 100, which is discussed in detail below with respect to FIGS. 2-7. Examples of airway adapters suitable for use with the present invention are taught in U.S. patent nos. 5,789,660; 6,312,389; and 7,174,789, the contents of which are incorporated herein by reference.

FIG. 2 depicts an electro-pneumatic assembly 100 suitable for use in a respiratory measurement system in accordance with the principles of the present invention. Electro-pneumatic assembly 100 comprises a housing assembly 200, a circuit board assembly 300, and a flow delivery component 285, which includes a pump support 290 and a pump 294. All of the components of the electro-pneumatic assembly are assembled prior to use. Other flow delivery components are contemplated for the electro-pneumatic assembly, including, for example, compressed gas sources. Upon assembly of housing assembly 200 and circuit board assembly 300, measurement and control components are in fluid communication with conduits and each other. As discussed in detail below, flow delivery component 285 is in fluid communication with a pressure chamber, which is in fluid communication with the tubing of an adapter in the patient's breathing circuit.

The present invention allows for a housing of unitary construction for use in a respiratory measurement system. This housing is easily assembled into a flow/pressure measurement system and incorporates all of the functions of a conventional flow/pressure measurement systems. The size, assembly complexity, and cost of the flow measurement system is significantly reduced, at least in part, as a result of integrating all of the tubing and the pressure vessel into a single low-cost housing and at the same time eliminating the need for individual unit balancing. Thus, the flow/pressure measurement system of the present invention maximizes the ease of manufacturability.

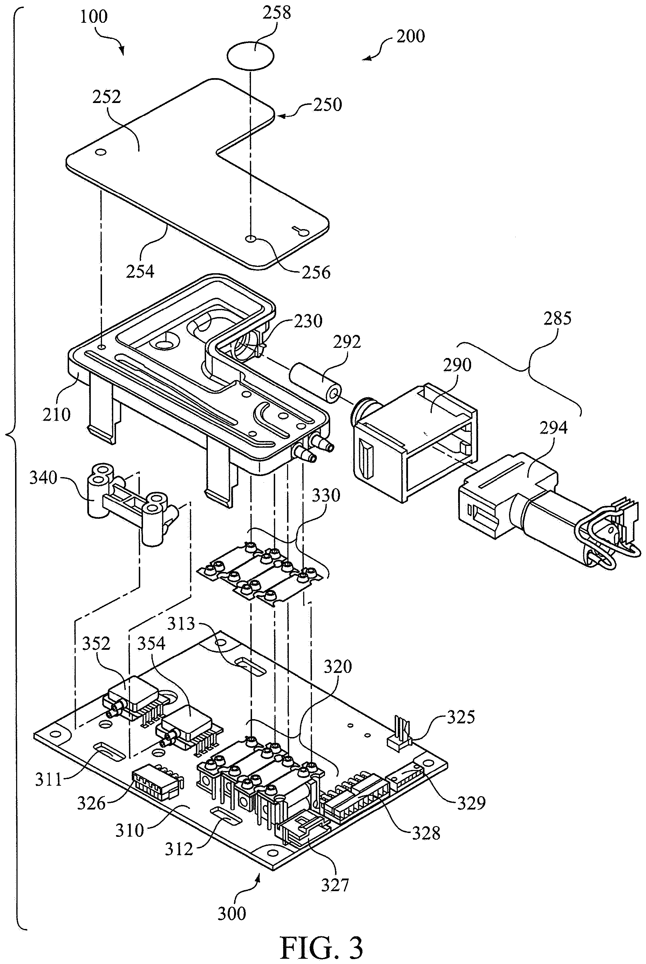

FIG. 3 depicts an exploded view of electro-pneumatic assembly 100. Housing assembly 200 comprises substantially rigid housing 210 and substantially planar cover 250. Housing 210 includes an upper surface 215 and a lower surface 225. Cover 250 includes a inner surface 254 and an outer surface 252. Housing 210 also includes a plurality of channels for transmitting pressure or flow, a cavity to serve as a flow/pressure reservoir, a plurality of openings in fluid communication with at least one channel, and (optionally) a plurality of openings in fluid communication with the cavity. Housing assembly 200 comprises all of the necessary pneumatics connections and volumes required in previous known respiratory flow/pressure measurement devices and provides all of these element in a "single" part.

As shown in FIGS. 3-7, inner surface 254 of housing cover 250 is affixed to the upper face of the housing to enclose a cavity 220, thereby forming a chamber. When the cover is closed over the housing, a plurality of conduits are also formed. With regard to the present invention, housing 210 and cover 250 of housing assembly 200 are made with sufficiently hard plastic to maintain dimensional stability and tight tolerances. Exemplary materials for housing 210 and cover 250 include thermoplastics such as ABS, polycarbonate (PC), PC/ABS blend, and acrylic.

The exemplary housing assembly 200 requires no tools to assemble onto circuit board 300 or to make pneumatic connections to the measurement and control components. Housing 210 and cover 250 are preferably joined by ultrasonic welding and preferably of the same material and of materials suitable for ultrasonic welding such as amorphous polymers. Alternative solutions include solvent bonding, epoxy, adhesive and the use of a die-cut label cut such that the channels would not be exposed to the adhesive.

The ultrasonic assembly process is dependent on the transmission of energy through thermoplastic parts to generate frictional heat at the joint area. The bonding with the cover is created using triangular structures in the housing known as energy directors that are molded onto the joint surfaces which are the exterior walls and interior walls between the channels. The primary purpose of the energy director is to "concentrate the energy to rapidly initiate the softening and melting of the joining surface." If joined by ultrasonic welding, the present invention contemplates molding the bonding surface of the cover with a textured surface to improve weld quality by increasing the friction between the parts.

Circuit board assembly 300 includes the measurement components, control components, interface components, and the associated electronics assembled onto circuit board 310. In the exemplary embodiment, the control components include valves 320, and measurement components include pressure transducers 352 and 354. Commercial examples of valves suitable for use in the present invention are two-way or three-way miniature solenoid valves available from Lee Company (Westbrook, CT) and Parker Pneutronics (Hollis, NH). The valves in the exemplary embodiment are 8 mm wide, 9 mm high and 24 mm long and include three ports to permit interfacing to tubing with a 1/16" inner diameter or a manifold. Pressure transducer 354 is configured to measure a differential pressure and pressure transducer 352 is configured to measure a gage pressure. Commercial examples of pressure transducers suitable for use in the present invention are available from Honeywell and All Sensors (Morgan Hill, CA).

Interface components include electrical connectors 325, 326, 327, 328 and 329. Electrical connector 325 interfaces with a connector portion of pump 294. Electrical connector 325 provides an interface for future expansion. Electrical connector 327 provides the interface for the optical detection. Electrical connector 328 provides the general I/O and power connection to a host system. The present invention contemplates interfacing electro-pneumatic assembly 100 to other monitoring components and computing derived parameters from the measurements made with these other components and electro-pneumatic assembly 100. As such, electrical connection 329 provides the connection for a separate monitoring component, which in an exemplary embodiment is the Capnostat 5 sensor or Quo sensor (both from Respironics, Inc). With the gas waveform data from these sensors, in combination with the flow and pressure measurements performed by the electro-pneumatic assembly 100, volumetric gas measurements, such oxygen consumption, carbon dioxide elimination, dead space, and slopes and angles from the volumetric gas waveforms, may be made using the processor on circuit board 310 or on a processor on host system. Circuit board 310 also includes slots 311, 312, and 313 to allow easy assembly of housing assembly 200 with circuit board 300.

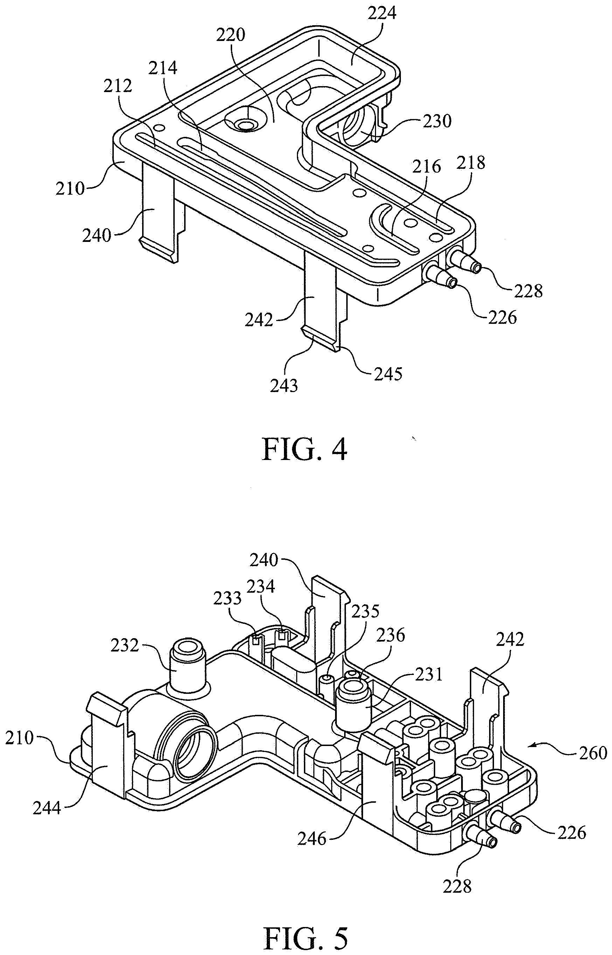

Also depicted in FIG. 3 are valve seals 330, transducer seal 340, pump filter 292, pump support 290, and pump 294. Valve seals 330 are press-fit to the ports of valves 320 and inner diameter of valve connection ports 260 (see FIG. 5) on the bottom surface of housing 210 to create a substantially leak-free connection between each valve port and valve connection port. Also shown is a transducer seal 340, which is press-fit as well, and serves to connect the two ports of pressure transducers 352 and 354 with the respective connection ports 233, 234 and 235, 236 (see FIG. 5). Exemplary materials for the transducer seal and valve seals include silicone, nitrile rubber, EPDM, and polyurethane. Note that while press fit assembly is preferred for ease of assembly; other methods of joining may be used such as gluing. Pump filter 292 is press-fit into pump support 290 and assembled pump support 290 with pump filter 292 is inserted into an opening 230 of housing 210. Exemplary materials for the pump filter include sintered polyethylene, porous metal, and sintered porous metal.

FIGS. 4 and 5 depict upper and lower perspective views of housing 210. Housing 210 includes snap latches 240, 242, 244, and 246, each of which includes a cantilever beam with a bump that deflects. To assemble housing assembly 200 with circuit board assembly 300, snap latches 240, 242, 244, 246 are snapped into slots 311, 312, and 313, respectively in circuit board 310 (note that a fourth slot that receives snap latch 246 is not shown in the figures. For illustrative purposes, bump 245 of snap latch 242 (as well as the other snap latches) includes a surface 243 forming a 90° hook that mates with slot 311, which also has a 90° recess with a window in the side, to allow snap latch 242 to be disengaged for disassembly with the application of a perpendicular force. Standoffs 231 and 232 are provided on housing 210 to provide structural stability during assembly and operation. Barb fittings 226 and 228 serve to interface electro-pneumatic assembly 100 to the input tubing and thereby serve as the input ports for the differential and airway pressure measurements. Channels 212, 214, 216 and 218 provide fluid connections between the measurement and control components.

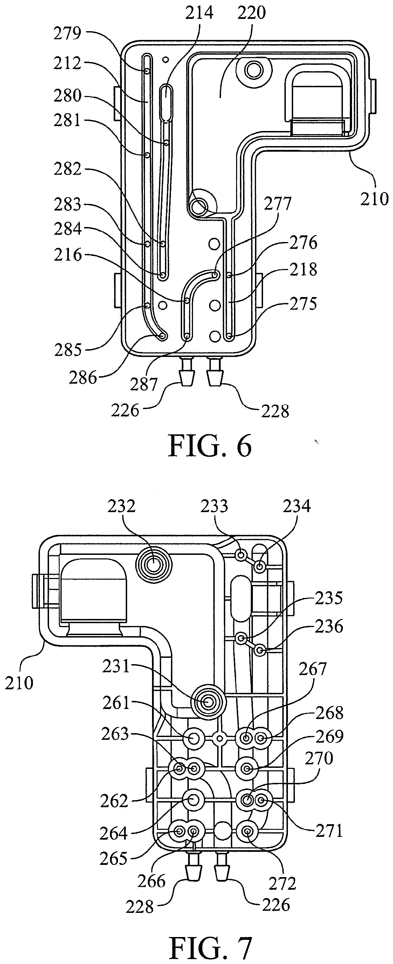

FIGS. 6 and 7 are the lower and upper views of housing 210 in which the openings and channels are clearly depicted. Connection ports 226 and 228 are in fluid communication with an airway adapter 20 in the breathing circuit of the patient. Connection port 226 is in fluid communication with the ventilator side (in the case of a mechanically ventilated patient) or atmospheric side (in the case of the spontaneously breathing patient) of the adapter and with channel 216 and its respective valve connection ports and openings. Connection port 228 is in fluid communication with the patient side of the adapter and with valve connection portion 266. Channel 212 includes openings 279, 281, 283, 285, and 286 which are in fluid communication with transducer connection ports 234 and 236, and valve connection ports 268, 271, and 272, respectively. Channel 214 includes openings 280, 282, and 284, which are in fluid communication with transducer connection port 235, and valve connection ports 267 and 269, respectively. Channel 216 includes openings 287 and 277, which are in fluid communication with connection port 226 and valve connection port 263. Channel 218 includes openings 276 and 275 and is in fluid communication with cavity 220. Openings 276 and 275 are in fluid communication with valve connection ports 262 and 265, respectively. Valve connection portions 233 and 234 are in fluid communications with the respective ports of pressure transducer 352. Valve connection portions 235 and 236 are in fluid communications with the respective ports of pressure transducer 354.

In the embodiment shown, all four valves are 3-way solenoid values. However, two of the valves function as a three-way valve and two function as a two-way valve. Connections/openings 261, 267, and 268 are associated with the first valve, which functions as a two way valve. Connections/openings 262, 263, and 269 are associated with second valve, which functions as a three-way valve. Connections/openings 264, 270, and 271 are associated with third valve, which functions as a two way valve. Connections/openings 265, 266, and 272 are associated with fourth valve which functions as a three-way valve. Also note that connections 261 and 264 are dead-ended. Opening 270 is in fluid communication with the atmospheric vent port 256. The present invention has (a) pneumatic manifold that is fully integrated with the purge system; (b) a smaller footprint; (c) shorter path lengths through the pneumatics to pressure transducers; and (d) novel method of assembly using methods such ultrasonic welding.

The exemplary electro-pneumatic assembly 100 shown in FIGS. 2-7 allows the measurement, zeroing, and purging functions required in a respiratory measurement system to be realized in a reliable cost-effective solution. The measurement function is performed by enabling fluid communication between pressure transducers 352 and 354 and connectors 226 and 228. The zeroing function is performed by enabling fluid communication between the ports of the pressure transducers and atmosphere and in the case of differential pressure transducer 354 enabling fluid communication between each port of the same pressure transducer as well.

Housing assembly 200 includes a volume, which is pressurized over a period of several seconds and the pressure released to enable a more effective purging function. This volume is formed by cavity 220 of housing 210 and a portion of cover 250. Cavity 220 includes a wall 224 and is enclosed by cover 250 to create chamber which serves as the pressure vessel. Pump 294, when actuated, delivers air into the chamber. The valves connected to the openings in channel 218 remain closed until the chamber is sufficiently pressurized. If the tubing on the ventilator side of the airway adapter is to be purged, then the valve opens the pathway between opening 276 and 277. If the tubing on the patient side of the airway adapter is to be purged, then the valve opens the pathway between opening 275 and valve connection portion 266.

The electro-pneumatic assembly of the present invention permits easy assembly, the steps of which are summarized below:
1. Join housing 210 and cover 250;
2. Press-fit the valve seals and transducer seals into the pneumatic ports on the bottom face of the manifold assembly;
3. Install pump filter 292 (press-fit) into pump support 290;
4. Push assembled pump support 290 with pump filter 292 into opening 230 of housing assembly 200;
5. Join (e.g., snap) circuit board 310 and "loaded" housing assembly 200 together;
6. Plug pump 294 into pump support 290;
7. Electrically connect pump 294 to circuit board connector 325;
8. Press an atmosphere vent filter 258 into opening 256 of housing assembly 200;

The advantages achieved by the present invention include:
(1) A single piece design containing all pneumatics, pneumatic connections, circuit board mounting connections, and pressure vessel for purge.
(2) Higher performance - less internal volume, no need for balancing, nearly immune from leaks associated with primarily tubing system.
(3) Low cost - Greater than order of magnitude decrease in per piece cost from machined manifold to injection molded design.
(4) Ease of manufacture - Design permits complete assembly of device in under 5 minutes.

It should be noted that while the present invention has been describe above as including both a flow and pressure sensing capability, the present invention contemplates that only one of these functions can be provided. In which case, the same configuration for the components can be used, but the unneeded element, such as opening and sensors, can be blocked or eliminated. Conversely, the present invention also contemplates providing additional functions in 100 electro-pneumatic assembly 100, such as temperature, humidity, pH, or other gas monitoring functions. Of course, more than one type of function can also be provided, such as multiple flow or pressure measurements. In which case, the number of ports, sensors, and channels is increased as appropriate.

Although the invention has been described in detail for the purpose of illustration based on what is currently considered to be the most practical and preferred embodiments, it is to be understood that such detail is solely for that purpose and that the invention is not limited to the disclosed embodiments, but, on the contrary, is intended to cover modifications and equivalent arrangements that are within the scope of the appended claims. For example, it is to be understood that the present invention contemplates that, to the extent possible, one or more features of any embodiment can be combined with one or more features of any other embodiment.

## Claims

1. An electro-pneumatic assembly (100) for use in a respiratory measurement system comprising:
(a) a housing (210) having a plurality of channels (212, 214, 216, 218) defined therein and a plurality of walls (224) forming a cavity (220), wherein the housing includes an upper face and a lower face, wherein a plurality of apertures are defined in the lower face of the housing, and where an aperture is defined in the housing and adapted to be in fluid communication with a source of pressure;
(b) a cover (250) having an inner surface and an outer surface, wherein the inner surface is affixed to the upper face of the housing to enclose the cavity thereby forming a chamber, and to enclose the channels thereby forming a plurality of conduits;
(c) a control component (320) operatively coupled to at least one aperture in the plurality of apertures and which comprises a valve;
(d) a measurement component (352, 354) operatively coupled to at least one aperture in the plurality of apertures, wherein the measurement component is a pressure sensor, flow sensor, or both a pressure sensor and a flow sensor;
(e) a flow delivery component (285) in fluid communication with the chamber and which comprises a pump (294); and
(f) a circuit board (310) **characterized in that** the control component, the measurement component, and the housing are affixed to the circuit board, and **in that** the housing (210) is assembled onto the circuit board (310), wherein upon said assembly, pneumatic connections are made between the control component and the associated at least one aperture of the housing and between the measurement component (352, 354) and the associated at least one aperture of the housing.

2. The electro-pneumatic assembly of claim 1, wherein the control component includes a plurality of valves, and wherein the measurement component includes a plurality of sensors.

3. The electro-pneumatic assembly of claim 1, wherein the cover is substantially planar.

4. The electro-pneumatic assembly of claim 1, wherein the housing is substantially rigid.

5. A method for assembling an electro-pneumatic assembly (100) for use in a respiratory measurement system comprising:
providing a housing (210) having a plurality of channels (212, 214, 216, 218) defined therein and a plurality of walls (224) forming a cavity (220), wherein the housing includes an upper face and a lower face, wherein a plurality of apertures are defined in the lower face of the housing, and wherein an aperture is defined in the housing and adapted to be in fluid communication with a source of pressure;
providing a cover (250) having an inner surface and an outer surface;
affixing the inner surface of the cover to the upper face of the housing to enclose the cavity thereby forming a chamber and to enclose the channels thereby forming a plurality of conduits;
coupling a control component (320) which comprises a valve to at least one aperture in the plurality of apertures;
coupling a measurement component (352, 354) to at least one aperture in the plurality of aperture, wherein the measurement component is a pressure sensor, flow sensor, or both a pressure sensor and a flow sensor; and
coupling a flow delivery component (285) in fluid communication with the chamber and which comprises a pump (294);
**characterized in that** the control component and the measurement component are provided on a circuit board, and the method comprises assembling the housing (210) onto the circuit board (310) and **in that** upon said assembly, pneumatic connections are made between the control component and the associated at least one aperture of the housing and between the measurement component (352, 354) and the associated at least one aperture of the housing.

## Patentansprüche

1. Elektropneumatische Anordnung (100) zur Verwendung in einem Atemmesssystem, umfassend:
(a) ein Gehäuse (210) mit einer Vielzahl von Kanälen (212, 214, 216, 218), die darin definiert sind, und einer Vielzahl von Wänden (224), die einen Hohlraum (220) bilden, wobei das Gehäuse eine obere Fläche und eine untere Fläche aufweist, wobei eine Vielzahl von Öffnungen in der unteren Fläche des Gehäuses definiert sind, und wobei eine Öffnung in dem Gehäuse definiert und angepasst ist, um in Fluidverbindung mit einer Druckquelle zu sein;
(b) eine Abdeckung (250) mit einer Innenfläche und einer Außenfläche, wobei die Innenfläche an der Oberseite des Gehäuses befestigt ist, um den Hohlraum zu umschließen und dadurch eine Kammer zu bilden, und um die Kanäle zu umschließen und dadurch eine Vielzahl von Leitungen zu bilden;
(c) eine Steuerkomponente (320), die mit mindestens einer Öffnung in der Vielzahl von Öffnungen wirkgekoppelt ist und ein Ventil umfasst;
(d) eine Messkomponente (352, 354), die mit mindestens einer Öffnung in der Vielzahl von Öffnungen wirkgekoppelt ist, wobei die Messkomponente ein Drucksensor, Strömungssensor oder sowohl ein Drucksensor als auch ein Strömungssensor ist;
(e) eine Strömungsabgabekomponente (285) in Fluidverbindung mit der Kammer, die eine Pumpe (294) umfasst; und
(f) eine Leiterplatte (310), **dadurch gekennzeichnet, dass** die Steuerkomponente, die Messkomponente und das Gehäuse an der Leiterplatte befestigt sind, und dass das Gehäuse (210) auf der Leiterplatte (310) montiert ist, wobei bei dieser Anordnung pneumatische Verbindungen zwischen der Steuerkomponente und der zugehörigen mindestens einen Öffnung des Gehäuses sowie zwischen der Messkomponente (352, 354) und der zugehörigen mindestens einen Öffnung des Gehäuses hergestellt werden.

2. Elektropneumatische Anordnung nach Anspruch 1, wobei die Steuerkomponente eine Vielzahl von Ventilen beinhaltet und wobei die Messkomponente eine Vielzahl von Sensoren beinhaltet.

3. Elektropneumatische Anordnung nach Anspruch 1, wobei die Abdeckung im Wesentlichen ebenflächig ist.

4. Elektropneumatische Anordnung nach Anspruch 1, wobei das Gehäuse im Wesentlichen starr ist.

5. Verfahren zur Montage einer elektropneumatischen Anordnung (100) zur Verwendung in einem Atemmesssystem, umfassend:
Bereitstellen eines Gehäuses (210) mit einer Vielzahl von Kanälen (212, 214, 216, 218), die darin definiert sind, und einer Vielzahl von Wänden (224), die einen Hohlraum (220) bilden, wobei das Gehäuse eine obere Fläche und eine untere Fläche aufweist, wobei eine Vielzahl von Öffnungen in der unteren Fläche des Gehäuses definiert sind, und wobei eine Öffnung in dem Gehäuse definiert und angepasst ist, um in Fluidverbindung mit einer Druckquelle zu sein;
Bereitstellen einer Abdeckung (250) mit einer Innenfläche und einer Außenfläche;
Befestigen der Innenfläche des Deckels an der Oberseite des Gehäuses, um den Hohlraum zu umschließen und dadurch eine Kammer zu bilden, und die Kanäle zu umschließen und dadurch eine Vielzahl von Leitungen zu bilden;
Koppeln einer Steuerkomponente (320), die ein Ventil mit mindestens einer Öffnung in der Vielzahl von Öffnungen umfasst;
Koppeln einer Messkomponente (352, 354) mit mindestens einer Öffnung in der Vielzahl von Öffnungen, wobei die Messkomponente ein Drucksensor, Strömungssensor oder sowohl ein Drucksensor als auch ein Strömungssensor ist; und
Koppeln einer Strömungsabgabekomponente (285) in Fluidverbindung mit der Kammer, und die eine Pumpe (294) umfasst;
**dadurch gekennzeichnet, dass** die Steuerkomponente und die Messkomponente auf einer Leiterplatte bereitbestellt sind, und das Verfahren das Montieren des Gehäuses (210) auf der Leiterplatte (310) umfasst, und dass bei dieser Montage pneumatische Verbindungen zwischen der Steuerkomponente und der zugehörigen mindestens einen Öffnung des Gehäuses sowie zwischen der Messkomponente (352, 354) und der zugehörigen mindestens einen Öffnung des Gehäuses hergestellt werden.

## Revendications

1. Ensemble électropneumatique (100) destiné à être utilisé dans un système de mesure respiratoire comprenant :
(a) un boîtier (210) comportant une pluralité de canaux (212, 214, 216, 218) définis dans celui-ci et une pluralité de parois (224) formant une cavité (220), le boîtier comprenant une face supérieure et une face inférieure, dans lequel une pluralité d'ouvertures sont définies dans la face inférieure du boîtier, et une ouverture est définie dans le boîtier et adaptée pour être en communication fluidique avec une source de pression ;
(b) un couvercle (250) ayant une surface intérieure et une surface extérieure, la surface intérieure étant fixée à la face supérieure du boîtier pour enfermer la cavité en formant ainsi une chambre, et pour enfermer les canaux en formant ainsi une pluralité de conduits ;
(c) un composant de commande (320) couplé de manière opérationnelle à au moins une ouverture dans la pluralité d'ouvertures et qui comprend une soupape ;
(d) un composant de mesure (352, 354) couplé de manière opérationnelle à au moins une ouverture dans la pluralité d'ouvertures, le composant de mesure étant un capteur de pression, un capteur d'écoulement, ou à la fois un capteur de pression et un capteur d'écoulement ;
(e) un composant de distribution d'écoulement (285) en communication fluidique avec la chambre et qui comprend une pompe (294) ; et
(f) une carte de circuit imprimé (310), **caractérisé en ce que** le composant de commande, le composant de mesure, et le boîtier sont fixés à la carte de circuit imprimé, et **en ce que** le boîtier (210) est assemblé sur la carte de circuit imprimé (310), dans lequel, sur ledit ensemble, des connexions pneumatiques sont réalisées entre le composant de commande et la au moins une ouverture associée du boîtier et entre le composant de mesure (352, 354) et la au moins une ouverture associée du boîtier.

2. Ensemble électropneumatique selon la revendication 1, dans lequel le composant de commande comprend une pluralité de soupapes, et dans lequel le composant de mesure comprend une pluralité de capteurs.

3. Ensemble électropneumatique selon la revendication 1, dans lequel le couvercle est sensiblement plan.

4. Assemblage électropneumatique selon la revendication 1, dans lequel le boîtier est sensiblement rigide.

5. Procédé d'assemblage d'un ensemble électropneumatique (100) destiné à être utilisé dans un système de mesure respiratoire comprenant de :
fournir un boîtier (210) ayant une pluralité de canaux (212, 214, 216, 218) définis dans celui-ci et une pluralité de parois (224) formant une cavité (220), dans lequel le boîtier comprend une face supérieure et une face inférieure, dans lequel une pluralité d'ouvertures sont définies dans la face inférieure du boîtier, et une ouverture étant définie dans le boîtier et adaptée pour être en communication fluidique avec une source de pression ;
fournir un couvercle (250) ayant une surface intérieure et une surface extérieure ;
fixer la surface intérieure du couvercle sur la face supérieure du boîtier pour enfermer la cavité en formant ainsi une chambre et pour enfermer les canaux en formant ainsi une pluralité de conduits ;
coupler un composant de commande (320) qui comprend une soupape à au moins une ouverture de la pluralité d'ouvertures ;
coupler un composant de mesure (352, 354) à au moins une ouverture de la pluralité d'ouvertures, le composant de mesure étant un capteur de pression, un capteur d'écoulement, ou à la fois un capteur de pression et un capteur d'écoulement ; et
coupler un composant de distribution d'écoulement (285) en communication fluidique avec la chambre et qui comprend une pompe (294) ;
**caractérisé en ce que** le composant de commande et le composant de mesure sont disposés sur une carte de circuit imprimé, et le procédé comprend l'assemblage du boîtier (210) sur la carte de circuit imprimé (310) et **en ce que**, sur ledit assemblage, des connexions pneumatiques sont réalisées entre le composant de commande et la au moins une ouverture associée du boîtier et entre le composant de mesure (352, 354) et la au moins une ouverture associée du boîtier.
